(19) Europäisches Patentamt / European Patent Office / Office européen des brevets

(11) **EP 3 765 116 B1**

(12) **EUROPÄISCHE PATENTSCHRIFT**

(45) Veröffentlichungstag und Bekanntmachung des Hinweises auf die Patenterteilung:
**24.04.2024   Patentblatt 2024/17**

(21) Anmeldenummer: **19712925.7**

(22) Anmeldetag: **14.03.2019**

(51) Internationale Patentklassifikation (IPC):
**A61M 1/16** (2006.01)      **A61M 1/34** (2006.01)

(52) Gemeinsame Patentklassifikation (CPC):
**A61M 1/1603; A61M 1/1613; A61M 1/3403; A61M 1/341;** A61M 2205/3334; A61M 2205/50

(86) Internationale Anmeldenummer:
**PCT/EP2019/056414**

(87) Internationale Veröffentlichungsnummer:
**WO 2019/175306 (19.09.2019 Gazette 2019/38)**

(54) **VERFAHREN ZUM EINSTELLEN EINER STEUER- ODER REGELVORRICHTUNG EINER BLUTBEHANDLUNGSVORRICHTUNG, UND VORRICHTUNGEN**

METHOD FOR ADJUSTING AN OPEN-LOOP OR CONTROL-LOOP CONTROL DEVICE OF A BLOOD TREATMENT DEVICE, AND DEVICES

PROCÉDÉ DE RÉGLAGE D'UN DISPOSITIF DE COMMANDE OU DE RÉGULATION D'UN DISPOSITIF DE TRAITEMENT DE SANG, ET DISPOSITIFS CORRESPONDANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.03.2018   DE 102018106254**

(43) Veröffentlichungstag der Anmeldung:
**20.01.2021   Patentblatt 2021/03**

(73) Patentinhaber: **Fresenius Medical Care Deutschland GmbH**
**61352 Bad Homburg (DE)**

(72) Erfinder: **KLEWINGHAUS, Juergen**
**61440 Oberursel (DE)**

(74) Vertreter: **Bobbert & Partner Patentanwälte PartmbB Postfach 1252 85422 Erding (DE)**

(56) Entgegenhaltungen:
WO-A1-03/028860      WO-A1-2013/144793
US-A1- 2016 121 037      US-B1- 6 602 424

**Beschreibung**

Die vorliegende Erfindung betrifft ein Verfahren zum Einstellen einer Steuer- oder Regelvorrichtung einer Blutbehandlungsvorrichtung für eine anstehende Blutbehandlungssitzung gemäß Anspruch 1. Sie betrifft zudem eine Blutbehandlungsvorrichtung gemäß Anspruch 5. Des Weiteren betrifft die vorliegende Erfindung ein digitales Speichermedium gemäß Anspruch 10, ein Computerprogramm-Produkt gemäß Anspruch 11 sowie ein Computerprogramm gemäß Anspruch 12.

Aus der Praxis sind Vorrichtungen zur extrakorporalen Behandlung von Blut bekannt. Ziel mancher der mit ihnen in der Praxis durchgeführten Blutbehandlungssitzungen ist es, bei Abschluss der Behandlungssitzung ein vorgegebenes Behandlungsziel erreicht zu haben. Es ist weiter regelmäßig vorgesehen, das zu Behandlungsbeginn vorgegebene Behandlungsziel innerhalb einer vorgegebenen Zeitdauer zu erreichen. Hierzu können zu Behandlungsbeginn Behandlungsparameter wie Pumpenflüsse geeignet eingestellt werden. Weiter können vorab Vorgaben zu Zielwerten, bestimmte Blutparameter und/oder die Behandlungsdauer betreffend, gemacht werden.

Durch Unterbrechungen der Behandlungssitzung, beispielsweise bedingt durch einen Beutelwechsel oder durch medizinische Untersuchungen des Patienten, für welche der Patient von der Behandlungsvorrichtung getrennt werden muss, kann es vorkommen, dass die Behandlungsdauer nachträglich erhöht werden muss, um das angestrebte Behandlungsziel zu erreichen. Alternativ sind komplexe Regelungen vorstellbar, welche mit hohem, insbesondere messtechnischem, Aufwand während der laufenden Behandlungssitzung auf vorab vorgenommene Einstellungen wirken, um das vorab angegebene oder angedachte Behandlungsziel auch im Falle von Unterbrechungen usw. zu erreichen.

WO 03/028860 A1, US 2016/121037 A1 und WO 2013/144793 offenbaren verschiedene bekannte Verfahren zum Einstellen einer Steuer- oder Regelvorrichtung einer Blutbehandlungsvorrichtung.

Eine Aufgabe der vorliegenden Erfindung ist es, ein weiteres Verfahren anzugeben, mittels welchem ein gewünschtes Behandlungsziel auch im Fall von Unterbrechungen erzielbar sein kann. Zudem soll eine Blutbehandlungsvorrichtung vorgeschlagen werden, die dafür geeignet ist, das Verfahren auszuführen. Des Weiteren sollen ein geeignetes digitales Speichermedium, ein geeignetes Computerprogramm-Produkt und ein geeignetes Computerprogramm zum Ausführen des Verfahrens angegeben werden.

Die erfindungsgemäße Aufgabe wird durch ein Verfahren mit den Merkmalen des Anspruchs 1 gelöst. Sie wird ferner gelöst durch eine Blutbehandlungsvorrichtung mit den Merkmalen des Anspruchs 5. Des Weiteren wird die erfindungsgemäße Aufgabe durch ein digitales Speichermedium mit den Merkmalen des Anspruchs 10, durch ein Computerprogramm-Produkt mit den Merkmalen des Anspruchs 11 sowie durch ein Computerprogramm mit den Merkmalen des Anspruchs 12 gelöst.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "programmiert" oder "konfiguriert" die Rede ist, so ist auch offenbart, diese Begriffe gegeneinander auszutauschen.

Die Angaben "oben" und "unten" sind hierin bei Zweifel des Fachmanns als absolute oder relative Raumangaben zu verstehen, welche sich auf die Ausrichtung des betreffenden Bauteils während seines üblichen Gebrauchs beziehen.

Das erfindungsgemäße Verfahren betrifft das Einstellen einer Steuer- oder Regelvorrichtung, die zum Steuern oder Regeln (kurz: Steuern) einer Blutbehandlungsvorrichtung konfiguriert ist. Die zu steuernde Blutbehandlungsvorrichtung weist eine Blutpumpe auf. Die Blutpumpe ist mit einem extrakorporalen Blutkreislauf verbindbar und ausgestaltet, um ein Fluid, insbesondere Blut, durch den extrakorporalen Blutkreislauf zu fördern.

Die zu steuernde Blutbehandlungsvorrichtung weist eine Dialysierflüssigkeitszulaufleitung und optional eine Dialysierflüssigkeitspumpe auf. Die optionale Dialysierflüssigkeitspumpe ist ausgestaltet, um im Betrieb der Blutbehandlungsvorrichtung Dialysierflüssigkeit durch die Dialysierflüssigkeitszulaufleitung zu fördern, insbesondere in Richtung zu einem Blutfilter.

Die zu steuernde Blutbehandlungsvorrichtung weist optional weiter eine Ultrafiltrationspumpe zum Erzielen einer Ultrafiltration und/oder eine Effluentpumpe zum Abführen von Effluent (u.a. verbrauchtem Dialysat) auf.

Die zu steuernde Blutbehandlungsvorrichtung weist eine Eingabevorrichtung auf, über welche ein Anwender, beispielsweise Klinikpersonal oder eine Ärztin, Flusswerte, z. B. Werte von Flussraten, die während einer anstehenden Blutbehandlungssitzung mittels der Blutpumpe und/oder der Dialysierflüssigkeitspumpe und/oder der Ultrafiltrationspumpe und/oder der Effluentpumpe erzielt oder bewirkt werden sollen, eingeben kann. Alternativ oder ergänzend zur Eingabe von Flusswerten kann die Eingabe eines Behandlungsziels erfolgen, welches mit Abschluss der anstehenden Blutbehandlungssitzung erreicht sein soll.

Für die Ableitung der einzustellenden Flussraten aus der Dosisvorgabe (ml/(kg x h)) kann die Gewichtsangabe des Patienten erforderlich sein. Diese kann an der Blutbehandlungsvorrichtung vom Anwender vor der Behandlung optional ebenfalls, z. B. über die Eingabevorrichtung, eingegeben werden.

Die zu steuernde Blutbehandlungsvorrichtung weist eine Einlesevorrichtung zum Einlesen und/oder Speichern der vom Anwender eingegebenen Flusswerte und/oder des vom Anwender eingegebenen Behandlungsziels auf.

Die zu steuernde Blutbehandlungsvorrichtung weist die o. g. Steuer- oder Regelvorrichtung auf. Letztere ist konfiguriert und/oder vorbereitet, um die Blutbehandlungsvorrichtung nach Beginn der anstehenden Blutbehandlungssitzung anhand von Flusswerten und/oder im Hinblick auf angestrebte Behandlungsziele zu steuern und/oder zu regeln.

Die zu steuernde Blutbehandlungsvorrichtung weist weiter eine Speichervorrichtung auf, in der ein Korrekturfaktor ($f_{korr}$) als Konstante hinterlegt ist.

Das erfindungsgemäße Verfahren umfasst folgende Schritte:

a) Bereitstellen, Ermitteln oder Auslesen des Korrekturfaktors von oder aus der Speichervorrichtung (alternativ: eines Vorhaltefaktors);

b) Korrigieren der vom Anwender eingegebenen Flusswerte und/oder des vom Anwender eingegebenen Behandlungsziels um den Korrekturfaktor. Die Korrektur erfolgt vorzugsweise vor Beginn der Behandlungssitzung. Die Korrektur erhöht vorzugsweise den eingestellten Flusswert und/oder das vom Anwender eingegebene Behandlungsziel (bzw. dessen Wert oder Höhe) um den Korrekturfaktor.

Die erfindungsgemäße Blutbehandlungsvorrichtung, die mit dem erfindungsgemäßen Verfahren gesteuert werden soll, weist eine Blutpumpe auf. Diese Blutpumpe ist mit einem extrakorporalen Blutkreislauf verbindbar und ausgestaltet, um ein Fluid, insbesondere Blut, durch den extrakorporalen Blutkreislauf zu fördern.

Die erfindungsgemäße Blutbehandlungsvorrichtung weist eine Dialysierflüssigkeitszulaufleitung und eine Dialysierflüssigkeitspumpe und/oder eine Ultrafiltrationspumpe und/oder eine Effluentpumpe auf. Die optionale Dialysierflüssigkeitspumpe ist ausgestaltet, um im Betrieb der Blutbehandlungsvorrichtung Dialysierflüssigkeit durch die Dialysierflüssigkeitszulaufleitung zu fördern, insbesondere in Richtung zu einem Blutfilter.

Die erfindungsgemäße Blutbehandlungsvorrichtung weist eine Eingabevorrichtung auf, über welche ein Anwender, beispielsweise Klinikpersonal oder eine Ärztin, Flusswerte, z. B. Werte von Flussraten, die während einer anstehenden Blutbehandlungssitzung mittels der Blutpumpe und/oder der Dialysierflüssigkeitspumpe und/oder der Ultrafiltrationspumpe und/oder der Effluentpumpe erzielt oder bewirkt werden sollen, eingeben kann. Alternativ oder ergänzend zur Eingabe von Flusswerten kann die Eingabe eines Behandlungsziels erfolgen, welches mit Abschluss der anstehenden Blutbehandlungssitzung erreicht sein soll.

Die erfindungsgemäße Blutbehandlungsvorrichtung weist eine Einlesevorrichtung zum Einlesen und/oder Speichern der vom Anwender eingegebenen Flusswerte und/oder des vom Anwender eingegebenen Behandlungsziels auf.

Die erfindungsgemäße Blutbehandlungsvorrichtung weist die oben genannte Steuer- oder Regelvorrichtung auf. Letztere ist konfiguriert und/oder vorbereitet, um die Blutbehandlungsvorrichtung nach Beginn der anstehenden Blutbehandlungssitzung anhand von Flusswerten und/oder im Hinblick auf angestrebte Behandlungsziele zu steuern und/oder zu regeln.

Die erfindungsgemäße Blutbehandlungsvorrichtung weist weiter eine Speichervorrichtung auf, in der ein Korrekturfaktor ($f_{korr}$) als Konstante hinterlegt ist.

Die erfindungsgemäße Blutbehandlungsvorrichtung ist geeignet, durch das erfindungsgemäße Verfahren gesteuert und/oder geregelt zu werden, wobei das Verfahren folgende Schritte umfasst:

a) Bereitstellen, Ermitteln oder Auslesen des Korrekturfaktors von oder aus der Speichervorrichtung (alternativ: eines Vorhaltefaktors);

b) Korrigieren der vom Anwender eingegebenen Flusswerte und/oder des vom Anwender eingegebenen Behandlungsziels um den Korrekturfaktor. Die Korrektur erfolgt vorzugsweise vor Beginn der Behandlungssitzung. Die Korrektur erhöht vorzugsweise den eingestellten Flusswert und/oder das vom Anwender eingegebene Behandlungsziel (bzw. dessen Wert oder Höhe) um den Korrekturfaktor.

Ein erfindungsgemäßes digitales, insbesondere nichtflüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Ein erfindungsgemäßes Computerprogramm-Produkt weist einen volatilen, flüchtigen oder auf einem maschinenlesbaren Träger gespeicherten Programmcode oder eine Signalwelle zur Veranlassung der maschinellen Schritte des erfindungsgemäßen Verfahrens auf, wenn das Computerprogramm-Produkt auf einem Rechner abläuft. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Server-System, Cloud Computing System, etc.) oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computerprogramm weist einen Programmcode zur Veranlassung der maschinellen Schritte des erfindungsgemäßen Verfahrens auf, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das erfindungsgemäße digitale Speichermedium, das erfindungsgemäße Computerprogramm-Produkt und das erfindungsgemäße Computerprogramm gilt, dass alle, einige oder manche der maschinell durchgeführten Schritte des erfindungsgemäßen Verfahrens veranlasst werden. Dies gilt insbesondere im Zusammenwirken mit einer Erfassungsvorrichtung und/oder einer erfindungsgemäßen Blutbehandlungsvorrichtung wie hierin beschrieben.

Erfindungsgemäße Ausführungsformen können eines oder mehrere der vorstehend oder im Folgenden genannten Merkmale aufweisen. Dabei können die hierin genannten Merkmale in beliebiger Kombination Gegenstand von erfindungsgemäßen Ausführungsformen sein, sofern der Fachmann eine konkrete Kombination nicht als technisch unmöglich erkennt.

Wann immer hierin von einer Ausführungsform die Rede ist, so handelt es sich um eine beispielhafte, erfindungsgemäße Ausführungsform.

Von einer Blutbehandlungssitzung ist in einigen Ausführungsformen erst die Rede, wenn der Patient mit der Blutbehandlungsvorrichtung, z. B. mittels des Blutschlauchsatzes, verbunden ist, wenn dem Patienten Blut entnommen wurde und/oder wenn dem Patienten Blut zurückgeführt wurde, nicht jedoch zuvor.

Der Korrekturfaktor kann ein Erfahrungswert sein, der sich aus der Praxis und den täglichen Routinen in der jeweiligen Klinik des Anwenders ergeben kann. So kann beispielsweise eine Wartezeit für einen Beutelwechsel dadurch unterschiedlich lang sein, da die zu ersetzenden Beutel in der einen Klinik über lange Wege herangeschafft werden müssen, während sie in der anderen Klinik in der Nähe des Geräts gelagert sind.

Der Korrekturfaktor kann durch die gezielte Analyse zurückliegender Blutbehandlungssitzungen desselben Behandlungsschemas, die in derselben Klinik oder Station durchgeführt wurden, ermittelt und z. B. in der Maschinensteuerung der Blutbehandlungsvorrichtung hinterlegt werden.

Solche Analysen zurückliegender Blutbehandlungssitzungen kann bei gewissen Blutbehandlungsvorrichtungen, insbesondere lernfähigen Blutbehandlungsvorrichtungen, automatisch von der Maschinensteuerung der Blutbehandlungsvorrichtung durchgeführt werden. Damit kann vorteilhafterweise ein verbesserter Korrekturfaktor erzielt und auch automatisch eingestellt oder dem Anwender zum Einstellen vorgeschlagen werden. Dabei können vor allem planmäßig anfallende Tätigkeiten wie Beutelwechsel in die Auswertung der Stillstände des Systems einbezogen werden, die eine Verringerung der tatsächlich verabreichten Dosis gegenüber der eingestellten Behandlungsdosis oder des angestrebten Behandlungsziels (Flüsse oder Dosiseinstellung) bewirkt haben.

In einigen Ausführungsformen wird der eingegebene Korrekturfaktor einer Plausibilitätsprüfung unterzogen, beispielsweise mittels der Einlesevorrichtung oder einer anderen hierzu programmierten Vorrichtung. So kann überprüft werden, ob ein vom Anwender eingegebener Korrekturfaktor bzw. dessen Höhe für den konkreten Anwendungsfall plausibel ist. Entsprechende Kriterien können hinterlegt sein, etwa in der Speichervorrichtung. Die Kriterien können sich in Abhängigkeit von der Art der Blutbehandlung, von deren initial eingestellter Dauer, vom konkreten Patienten, von der Zugehörigkeit des Patienten zu einer Klasse von Patienten (Nebenerkrankungen, Alter, Mobilität, usw.) usw. voneinander unterscheiden. Die Plausibilitätsprüfung gilt beispielsweise als bestanden, wenn der Korrekturfaktor, ggf. bezogen auf den konkreten Patienten oder die Patientenklasse, dem Kriterium oder den Kriterien genügt.

In manchen nicht beanspruchten Ausführungsformen wird anstelle des Korrekturfaktors ein Vorhaltefaktor eingegeben und anstelle des Korrekturfaktors verwendet. Alles hierin zum Korrekturfaktor Ausgeführte gilt unverändert auch für den Vorhaltefaktor.

In einigen Ausführungsformen wird ein Wertebereich (beidseitig beschränkt oder einseitig beschränkt, etwa mittels Schwellenwert) eingegeben oder vorgegeben, innerhalb welchem der eingegebene Korrekturfaktor zu liegen hat. Sollte der Anwender Eingaben machen wollen, die außerhalb dieses Wertebereichs liegen, so kann die Einlesevorrichtung oder eine andere hierzu programmierte Vorrichtung dies unterbinden und/oder eine Anzeige oder Warnung an den Anwender ausgeben.

In manchen Ausführungsformen weist die Steuer- und Regelvorrichtung eine Routine zur Plausibilitätskontrolle des Korrekturfaktors auf, die dazu dient, die Eingabe nicht sinnvoller Werte gar nicht erst zuzulassen, beispielsweise durch die Angaben oberer und/oder unterer Grenzwerte oder eines Wertebereichs für den Korrekturfaktor, jenseits welcher eine Eingabe nicht möglich ist.

In manchen Ausführungsformen errechnet eine Vorrichtung - z. B. die Steuer- und/oder Regelvorrichtung - den Korrekturfaktor oder passt einen eingegebenen Korrekturfaktor automatisch an. Dazu kann sie beispielsweise relevante Pumpenwerte, die Summe der zu Behandlungsbeginn bereits bekannten späteren Abwesenheiten, z. B. die vorübergehende Abwesenheit des Patienten wegen geplanter medizinischer Untersuchungen, Sollfluss während der Ausfallzeiten oder die zu erwartende Laufzeit der Pumpen usw. heranziehen. Sie kann hierfür, insbesondere automatisch, ergänzend oder alternativ auf Werte, Zeiten und/oder wenigstens eine Beobachtung aus früheren Behandlungssitzungen desselben Patienten oder eines Kollektivs von Patienten zurückgreifen.

In einigen Ausführungsformen erfolgt das erfindungsgemäße Verfahren nicht nach Beginn der Behandlungssitzung.

In einigen Ausführungsformen ist das Behandlungsziel ein Behandlungsergebnis bezogen auf die Behandlungsdauer.

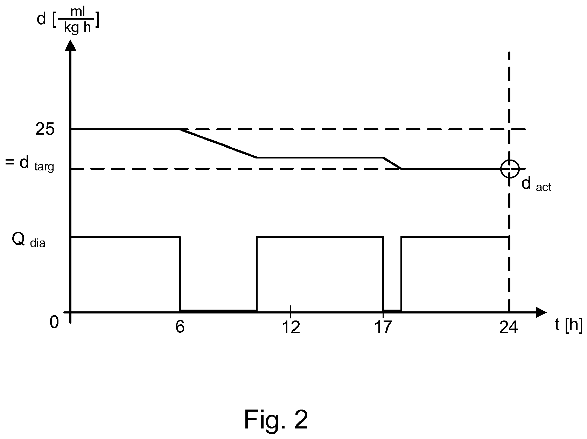

Ein Beispiel eines Behandlungsergebnisses ist die Behandlungsdosis bei der Dialyse oder die Dialysedosis, die als Wert mit der Dimension [ml/kg*h] angegeben werden kann, siehe auch Fig. 2.

Der Korrekturfaktor des erfindungsgemäßen Verfahrens ist als Konstante in der Speichervorrichtung hinterlegt und kann von dieser bereitgestellt und/oder aus dieser ausgelesen werden.

In einigen Ausführungsformen muss der in der Speichervorrichtung als Konstante hinterlegte Korrekturfaktor von speziell autorisierten Personen, beispielsweise autorisiertem Klinikpersonal und/oder einem autorisierten Techniker, angepasst werden, sollten Änderungen hieran gewünscht sein. Der Anwender ist in diesen Ausführungsformen hierzu nicht in der Lage oder nicht berechtigt.

In gewissen Ausführungsformen kann der Korrekturfaktor oder Kontrollwert bzw. die Konstante auf Erfahrungswerten basieren, wie oben bereits ausgeführt.

In manchen Ausführungsformen kann der Korrekturfaktor oder Kontrollwert aufgrund wahrscheinlicher und/oder planmäßiger Unterbrechungen im Blutbehandlungsablauf berechnet werden oder berechnet worden sein.

In gewissen Ausführungsformen, insbesondere im Rahmen extrakorporaler Blutbehandlungsverfahren mit regionaler Citrat-Antikoagulation, kann sich der Korrekturfaktor über die Blutpumpenrate auch auf eine Citratrate auswirken, wenn eine Dosierung einer Citratlösung automatisch und proportional zur Blutflussrate erfolgt.

Des Weiteren kann sich in manchen Ausführungsformen die Anwendung des Korrekturfaktors auf die Dialysatrate indirekt auch auf die Calciumrate auswirken, insbesondere dann, wenn diese wie üblich von der Effluentrate abhängt. Die Effluentrate ist in der Regel von der Netto-Ultrafiltrationsrate und außerdem von der Dialysatrate und Citratrate abhängig. Speziell für die Wirkweise der regionalen Citrat-Antikoagulation ist es vorteilhaft, dass der Korrekturfaktor auf beide Parameter (Blut- und Dialysatrate) gleichermaßen angewendet wird, um deren Verhältnis zueinander nicht zu verändern. Andernfalls könnte eine unbeabsichtigte Verschiebung des Säure-Basen-Status des Patienten erfolgen.

Die Anpassung, insbesondere Erhöhung, der Netto-Ultrafiltrationsrate oder Ultrafiltrationsrate mittels der Ultrafiltrationspumpe sollte vorzugsweise ebenfalls mittels des Korrekturfaktors erfolgen. Dies kann in manchen erfindungsgemäßen Ausführungsformen vorgesehen sein.

In gewissen Ausführungsformen kann über die Anwendung des Korrekturfaktors auf die Dialysatrate indirekt auch eine Erhöhung der Effluentrate erfolgen, da hiermit auch das einströmende Dialysat abgefördert wird.

In einigen Ausführungsformen beträgt der Korrekturfaktor einen Wert zwischen 10 % und 30 %, insbesondere zwischen 15 % und 25 %, ganz insbesondere bei 20 %. Alternativ oder ergänzend liegt die Korrektur der eingegebenen Flusswerte und/oder des vom Anwender eingegebenen Behandlungsziels in einem Bereich zwischen 10 % und 30 %, insbesondere zwischen 15 % und 25 %, ganz insbesondere bei 20 %. Die vorstehenden Prozentangaben beziehen sich auf wenigstens einen, mehrere (beliebige und beliebige Kombinationen) oder alle vom Anwender eingegebenen Flusswerte und/oder auf das vom Anwender eingegebene Behandlungsziel (bzw. dessen Wert oder Höhe).

In manchen Ausführungsformen wird oder wurde der Korrekturwert des erfindungsgemäßen Verfahrens mittels der Eingabevorrichtung vom Anwender, beispielsweise Ärztin oder Klinikpersonal, eingegeben.

In gewissen Ausführungsformen ist die Blutbehandlungsvorrichtung als Hämodialysevorrichtung, Hämofiltrationsvorrichtung oder als Hämodiafiltrationsvorrichtung ausgestaltet, insbesondere als eine Vorrichtung für die chronische Nierenersatztherapie oder für die kontinuierliche Nierenersatztherapie (CRRT = *continuous renal replacement therapy*).

Manche oder alle erfindungsgemäßen Ausführungsformen können einen, mehrere oder alle der oben und/oder im Folgenden genannten Vorteile aufweisen.

Mit Hilfe des erfindungsgemäßen Verfahrens lässt sich vorteilhafterweise ein erhöhter messtechnischer Aufwand unter Verwendung von komplexer Sensortechnik zur Regelung und Steuerung von Pumpensystemen der Blutbehandlungsvorrichtung vermeiden. Dieser Aufwand wäre alternativ zum hierin beschriebenen Vorgehen notwendig, wollte man die Blutbehandlungsvorrichtung in die Lage versetzen, ein Behandlungsziel selbst dann zu erreichen, wenn die Blutbehandlungssitzung unterbrochen werden sollte. Die Steuervorrichtung könnte einen Soll/Ist-Abgleich zwischen bis zur Unterbrechung Erreichtem und bis zum Behandlungssitzungsende Angestrebtem vornehmen und die Blutbehandlungsvorrichtung nach Ende der Unterbrechung entsprechend regeln (was erfindungsgemäß nicht erforderlich ist und in einigen Ausführungsformen daher unterbleibt). Hierzu bedarf es jedoch eines vergleichsweise hohen messtechnischen Aufwands. Dieser messtechnische Aufwand resultierte bisher in höheren Hardwarekosten und in einem erhöhten Validierungsaufwand für die Gerätesoftware. Mit dem erfindungsgemäßen Verfahren lassen sich der hierzu nötige Aufwand und die hiermit verbundenen Kosten vorteilhaft reduzieren oder vermeiden.

Da das erfindungsgemäße Verfahren einfach zu realisieren und auch einfach, beispielsweise durch ein einfaches Softwareupdate, zu installieren ist, bietet sich vorteilhaft auch die Nachrüstung bestehender Geräte an.

Vorteilhafterweise wird im Rahmen des erfindungsgemäßen Verfahrens auf fehleranfällige Sensortechnik zur Kontrolle der Korrektur verzichtet, was einen einfacheren und wartungsfreieren Betrieb der Blutbehandlungsvorrichtung und/oder Ablauf der Blutbehandlung zur Folge haben kann.

Ein weiterer Vorteil des erfindungsgemäßen Verfahrens ist, dass seine Automatisierung nicht zulässt, dass der Anwender Schritte, die zur Kompensierung des Behandlungsrückstands aufgrund von Unterbrechungen der Behandlungssitzung erforderlich sind, vergessen kann.

Außerdem können bei der Korrektur, insbesondere durch die Verwendung vorbestimmter und ggf. auch bereits auf Plausibilität überprüfter Kontrollfaktoren, keine Berechnungsfehler im Laufe der Behandlung vorkommen, was wiederum die Sicherheit für den Patienten erhöht.

Schließlich muss sich der Anwender vorteilhafterweise keine Gedanken über eine Korrektur machen. Es kann für ihn genügen, z. B. das Behandlungsziel in der ihm vertrauten Größe einzustellen. Etwaig erforderliche Korrekturen kann ihm die Steuervorrichtung abnehmen, beispielsweise durch Auslesen, Ermitteln und/oder Berücksichtigen des Korrekturfaktors, vermutlich sogar qualitativ hochwertiger als ein Anwender dies könnte.

Die vorliegende Erfindung wird im Folgenden anhand der beigefügten Zeichnung, in welcher gleiche Bezugszeichen gleiche oder ähnliche Bauteile bezeichnen, exemplarisch erläutert. In den Figuren gilt:

**Fig. 1** zeigt in vereinfachter Darstellung eine erfindungsgemäße Blutbehandlungsvorrichtung mit einem extrakorporalen Blutkreislauf in einer Ausführungsform;

**Fig. 2** zeigt exemplarisch in graphischer Darstellung den 24stündigen Verlauf einer Blutbehandlungssitzung ohne Verwendung des erfindungsgemäßen Verfahrens;

**Fig. 3** zeigt in graphischer Darstellung den 24stündigen Verlauf einer Blutbehandlungssitzung mit einer exemplarischen Ausführungsform des erfindungsgemäßen Verfahrens; und

**Fig. 4** zeigt den Ablauf einer exemplarischen Ausführungsform des erfindungsgemäßen Verfahrens in einem Flussdiagramm.

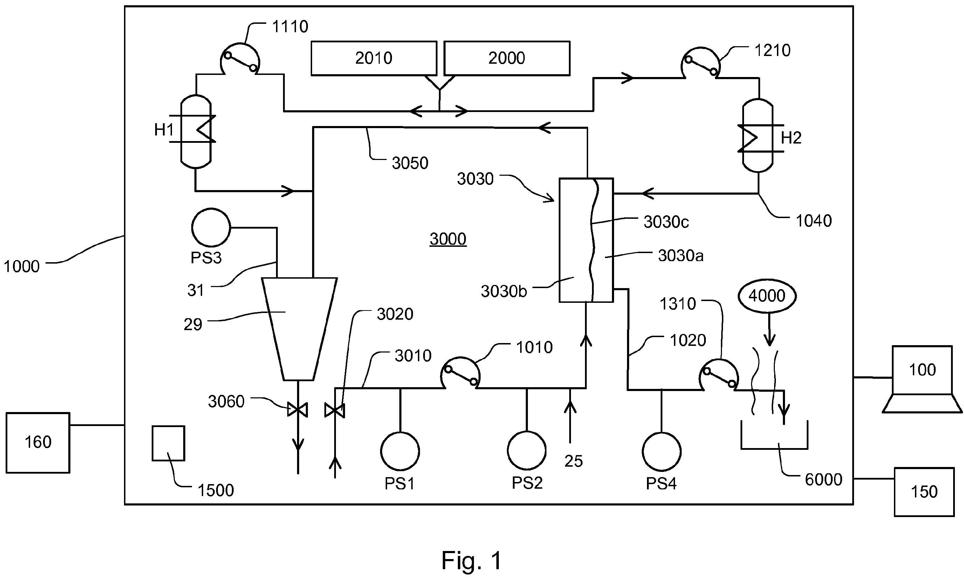

**Fig. 1** zeigt in stark vereinfachter Darstellung eine erfindungsgemäße Blutbehandlungsvorrichtung 1000, verbunden mit einem extrakorporalen Blutkreislauf 3000 und einem nur angedeuteten erfindungsgemäßen Abflussschlauchsystem mit einem optionalen Effluentbeutel 4000. Der extrakorporale Blutkreislauf 3000 weist eine erste Leitung 3010, hier in Form eines arteriellen Leitungsabschnitts, auf.

Die erste Leitung 3010 steht in Fluidverbindung mit einer Blutbehandlungseinrichtung, hier exemplarisch ein Blutfilter oder Dialysator 3030. Der Blutfilter 3030 weist eine Dialysierflüssigkeitskammer 3030a und eine Blutkammer 3030b auf, welche durch eine zumeist semi-permeable Membran 3030c voneinander getrennt sind.

Der extrakorporale Blutkreislauf 3000 weist ferner wenigstens eine zweite Leitung 3050, hier in Form eines venösen Leitungsabschnitts oder einer Rückgabeleitung, auf. Sowohl die erste Leitung 3010 als auch die zweite Leitung 3050 können zur Verbindung mit dem Gefäßsystem des nicht dargestellten Patienten dienen.

Die erste Leitung 3010 ist optional mit einer (ersten) Schlauchklemme 3020 zum Sperren oder Schließen der Leitung 3010 verbunden. Die zweite Leitung 3050 ist optional mit einer (zweiten) Schlauchklemme 3060 zum Sperren oder Schließen der Leitung 3050 verbunden.

Die in Fig. 1 nur durch einige ihrer Einrichtungen und schematisch repräsentierte Blutbehandlungsvorrichtung 1000 weist eine Blutpumpe 1010 auf. Die Blutpumpe 1010 fördert während der Behandlung des Patienten Blut durch Abschnitte des extrakorporalen Blutkreislaufs 3000 und in Richtung zum Blutfilter oder Dialysator 3030, wie die kleinen Pfeilspitzen, welche in der Fig. 1 allgemein die Strömungsrichtung angeben, zeigen.

Mittels einer Pumpe 1210 für Dialysierflüssigkeit, die als Rollenpumpe oder als anderweitig okkludierende Pumpe ausgestaltet sein kann, wird frische Dialysierflüssigkeit aus einer Quelle 2000 entlang der Dialysierflüssigkeitszulaufleitung 1040 in die Dialysierflüssigkeitskammer 3030a gepumpt. Die Dialysierflüssigkeit verlässt die Dialysierflüssigkeitskammer 3030a als Dialysat, ggf. angereichert durch Filtrat, in Richtung des Ausgusses 6000 und wird hierin als Effluent bezeichnet.

Die Quelle 2000 kann beispielsweise ein Beutel oder ein Container sein. Die Quelle 2000 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung 1000.

Eine weitere Quelle 2010 mit Substituat kann optional vorgesehen sein. Sie kann der Quelle 2000 entsprechen oder eine eigene Quelle sein.

Rechts unten ist in Fig. 1 angedeutet, wo das Abflussschlauchsystem mit dem Effluentbeutel 4000 mit der Blutbehandlungsvorrichtung 1000 verbunden ist.

Neben der vorgenannten Blutpumpe 1010 weist die in Fig. 1 gezeigte Anordnung ferner rein optional eine Reihe weiterer, jeweils optionaler Pumpen, nämlich die Pumpe 1110 für Substituat, die Pumpe 1210 für Dialysierflüssigkeit, und die Pumpe 1310 für das Effluent auf.

Die Pumpe 1210 ist vorgesehen, um Dialysierflüssigkeit aus einer Quelle 2000, beispielsweise einem Beutel, heraus und über eine optional vorhandene Beutelheizung mit einem Beutel H2 dem Blutfilter 3030 mittels einer Dialysierflüssigkeitszulaufleitung 1040 zuzuführen.

Die auf diese Weise zugeführte Dialysierflüssigkeit tritt über eine Dialysatablaufleitung 1020, unterstützt durch die Pumpe 1310, wieder aus dem Blutfilter 3030 aus und kann verworfen werden.

Stromauf der Blutpumpe 1010 ist ein optionaler arterieller Sensor PS1 vorgesehen. Während einer Behandlung des Patienten misst er den Druck in der arteriellen Leitung.

Stromab der Blutpumpe 1010, jedoch stromauf des Blutfilters 3030 und, falls vorgesehen, einer Zugabestelle 25 für Heparin, ist ein weiterer, optionaler Drucksensor PS2 vorgesehen. Er misst den Druck stromauf des Blutfilters 3030 ("prä-Hämofilter").

Ein wiederum weiterer Drucksensor kann als PS4 stromab des Blutfilters 3030, jedoch vorzugsweise stromauf der Pumpe 1310, in der Dialysatablaufleitung 1020 zum Messen des Filtratdrucks des Blutfilters 3030 vorgesehen sein.

Blut, das den Blutfilter 3030 verlässt, durchströmt eine optionale venöse Blutkammer 29, welche eine Entlüftungseinrichtung 31 aufweisen und mit einem weiteren Drucksensor PS3 in Fluidverbindung stehen kann.

Eine Steuer- oder Regelvorrichtung 1500 zum Steuern oder Regeln der Blutbehandlungsvorrichtung 1000 kann vorgesehen sein und mit allen o. g. Komponenten der Blutbehandlungsvorrichtung 1000 in Signal- und/oder Steuerverbindung stehen.

Ferner sind mit der Blutbehandlungsvorrichtung 1000 eine Eingabevorrichtung 100, eine Einlesevorrichtung 150 und eine Speichervorrichtung 160 in Signal- und/oder Steuerkommunikation verbunden.

**Fig. 2** zeigt ein Behandlungsergebnis d (in [ml/kg*h]) über der Zeit t (in [h]) bei Ablauf einer herkömmlichen, auf 24 Stunden festgesetzten Blutbehandlungssitzung zur akuten Behandlung des Patienten mit zwei Unterbrechungen, welche nach 6 Stunden bzw. nach 17 Stunden jeweils beginnen. Die Unterbrechungen sind jeweils am Abfall von $Q_{dia}$ zu erkennen. $Q_{dia}$ entspricht dem Fluss der Dialysierflüssigkeitspumpe 1210. Andere Pumpenflüsse, etwa $Q_B$ für die Blutpumpe 1010, $Q_{UF}$ für die Ultrafiltrationspumpe oder Flüsse der Effluentpumpe können ebenfalls von den Unterbrechungen betroffen sein.

Von den beiden Unterbrechungen ist auch das Behandlungsergebnis betroffen: d sinkt bei und während jeder Unterbrechung weiter ab. Es würde eines Regelungsmechanismus bedürfen, um bis zum Ablauf der vorgegebenen 24 Stunden auf das gewünschte Behandlungsergebnis $d_{targ}$ von 25 ml/kg*h zu kommen.

Wie Fig. 2 zu entnehmen ist, wird das eingestellte Behandlungsziel $d_{targ}$ aufgrund der beiden Unterbrechungen nach 24 Stunden nicht erreicht, da das tatsächlich erreichte Behandlungsergebnis $d_{act}$ kleiner als $d_{targ}$ ist.

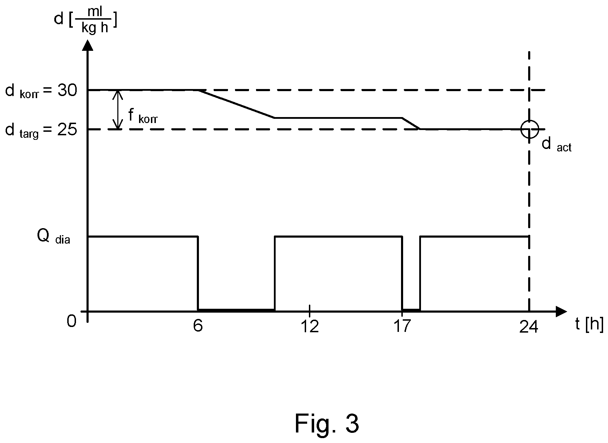

**Fig. 3** zeigt den Ablauf einer Blutbehandlungssitzung mit denselben Unterbrechungen wie in Fig. 2, hier allerdings unter Verwenden einer exemplarischen Ausführungsform des erfindungsgemäßen Verfahrens. Das Behandlungsziel wurde bereits vor Beginn der Behandlungssitzung, also bei t kleiner oder gleich 0, mittels eines Korrekturfaktors $f_{korr}$ (hier exemplarisch um 20 %) automatisch nach oben korrigiert. Pumpenflüsse und/oder andere Maschinenparameter wurden auf das neue, höhere, korrigierte Behandlungsziel $d_{korr}$ angehoben. Hiervon ausgehend wird nach 24 Stunden das eingestellte, ursprüngliche Behandlungsziel erreicht, es gilt $d_{targ} = d_{act}$.

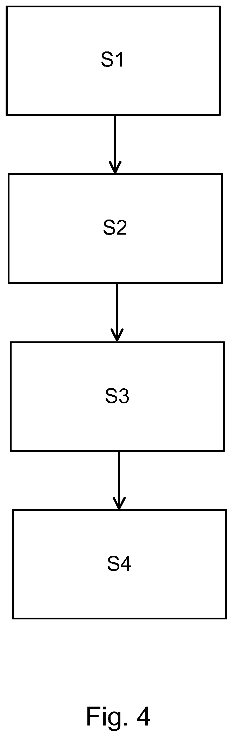

**Fig 4** zeigt den Ablauf einer exemplarischen Ausführungsform des erfindungsgemäßen Verfahrens in einem Flussdiagramm.

Hierbei beschreibt S1 die Eingabe des Korrekturfaktors $f_{korr}$ bzw. das Einlesen desselben aus der Speichervorrichtung 160.

Der Schritt S2 beschreibt das Setzen des Anfangswerts $d_{korr}$ unter Berücksichtigung des Korrekturfaktors $f_{korr}$,

addiert zum Behandlungsziel $d_{targ}$.

Schritt S3 steht für die laufende Behandlung über 24 Stunden hinweg, mit Unterbrechungen der Behandlungssitzung.

Schritt S4 steht für das Erreichen des Behandlungsziels $d_{targ}$ am Ende der 24stündigen Behandlungssitzung, wobei vorzugsweise $d_{act} = d_{targ}$ erreicht wird.

**Bezugszeichenliste**

| 25 | Zugabestelle für Heparin (optional) |
| 29 | venöse Blutkammer |
| 31 | Entlüftungseinrichtung |

| 100 | Eingabevorrichtung |
| 150 | Einlesevorrichtung |
| 160 | Speichervorrichtung |

| 1000 | Blutbehandlungsvorrichtung |
| 1010 | Blutpumpe |
| 1020 | Dialysatablaufleitung, Effluentzulaufleitung |
| 1040 | Dialysierflüssigkeitszulaufleitung |
| 1110 | Pumpe für Substituat |
| 1210 | Dialysierflüssigkeitspumpe |
| 1310 | Pumpe für Dialysat oder Effluent |
| 1500 | Steuer- oder Regelvorrichtung |

| 2000 | Quelle mit Dialysierflüssigkeit |
| 2010 | Quelle mit Substituat, optional |

| 3000 | extrakorporaler Blutkreislauf |
| 3010 | erste Leitung (arterieller Leitungsabschnitt) |
| 3020 | (erste) Schlauchklemme |
| 3030 | Blutfilter oder Dialysator |
| 3030a | Dialysierflüssigkeitskammer |
| 3030b | Blutkammer |
| 3030c | semi-permeable Membran |
| 3050 | zweite Leitung (venöser Leitungsabschnitt) |
| 3060 | (zweite) Schlauchklemme |

| 4000 | Effluentbeutel |
| 6000 | Ausguss |

| H2 | Beutelheizung mit Beutel (Dialysierflüssigkeit) |
| H1 | Beutelheizung mit Beutel (Substituat) |

| PS1, PS2 | arterieller Drucksensor (optional) |
| PS3 | venöser Drucksensor (optional) |
| PS4 | Drucksensor zum Messen des Filtratdrucks |

| $d_{act}$ | tatsächlich erreichtes Behandlungsergebnis |
| $d_{targ}$ | eingestelltes Behandlungsziel |
| $d_{korr}$ | korrigiertes Behandlungsziel |

| $f_{korr}$ | Korrekturfaktor, Vorhaltefaktor |
| $Q_{dia}$ | Fluss der Dialysierflüssigkeitspumpe |
| $Q_B$ | Fluss der Blutpumpe |
| $Q_{UF}$ | Fluss der Ultrafiltrationspumpe |

**Patentansprüche**

1. Verfahren zum Einstellen einer Steuer- oder Regelvorrichtung (1500) einer Blutbehandlungsvorrichtung (1000)für eine anstehende Blutbehandlungssitzung, wobei die Blutbehandlungsvorrichtung aufweist

   - eine Blutpumpe (1010), verbindbar mit einem extrakorporalen Blutkreislauf (3000) zum Fördern von Blut durch den extrakorporalen Blutkreislauf (3000);
   - eine Dialysierflüssigkeitszulaufleitung (1040);
   - eine Dialysierflüssigkeitspumpe (1210) zum Fördern von Dialysierflüssigkeit durch die Dialysierflüssigkeitszulaufleitung (1040) und/oder eine Ultrafiltrationspumpe und/oder eine Effluentpumpe;
   - eine Eingabevorrichtung (100) für den Anwender zum Eingeben von Flusswerten ($Q_B$, $Q_{dia}$, $Q_{UF}$), die während einer anstehenden Blutbehandlungssitzung mittels der Blutpumpe (1010) und/oder der Dialysierflüssigkeitspumpe (1210) und/oder der Ultrafiltrationspumpe und/oder der Effluentpumpe erzielt oder bewirkt werden sollen, und/oder zum Eingeben eines Behandlungsziels ($d_{targ}$), welches bei Abschluss der anstehenden Blutbehandlungssitzung erzielt sein soll;
   - eine Einlesevorrichtung (150) zum Einlesen und/oder Speichern der vom Anwender eingegebenen Flusswerte ($Q_B$, $Q_{dia}$, $Q_{UF}$) und/oder des vom Anwender eingegebenen Behandlungsziels ($d_{targ}$) ;
   - eine Steuer- oder Regelvorrichtung (1500), konfiguriert zum Steuern oder Regeln der Blutbehandlungsvorrichtung (1000) nach Beginn der anstehenden Blutbehandlungssitzung anhand von Flusswerten ($Q_B$, $Q_{dia}$, $Q_{UF}$) und/oder Behandlungszielen ($d_{targ}$) ;
   - eine Speichervorrichtung (160), in der ein Korrekturfaktor ($f_{korr}$) als Konstante hinterlegt ist;
   wobei das Verfahren folgende Schritte umfasst:

   a) Bereitstellen, Ermitteln oder Auslesen des Korrekturfaktors ($f_{korr}$) von oder aus der Speichervorrichtung (160);
   b) Korrigieren der vom Anwender eingegebenen Flusswerte ($Q_B$, $Q_{dia}$, $Q_{UF}$) und/oder des vom Anwender eingegebenen Behandlungsziels ($d_{targ}$) um den Korrekturfaktor ($f_{korr}$).

2. Verfahren nach Anspruch 1, wobei das Behandlungsziel ($d_{targ}$) ein Behandlungsergebnis bezogen auf die Behandlungsdauer ist.

3. Verfahren nach einem der vorangegangenen Ansprüche, wobei der Korrekturfaktor ($f_{korr}$) einen Wert zwischen 10 % und 30 %, insbesondere 20 % beträgt und/oder eine Korrektur der eingegebenen Flusswerte ($Q_B$, $Q_{dia}$, $Q_{UF}$) und/oder des vom Anwender eingegebenen Behandlungsziels ($d_{targ}$) zwischen 10 % und 30 %, insbesondere 20 % ergibt.

4. Verfahren nach einem der vorangegangenen Ansprüche, wobei der Korrekturfaktor ($f_{korr}$) mittels der Eingabevorrichtung (100) vom Anwender eingegeben wird oder wurde.

5. Blutbehandlungsvorrichtung (1000), welche aufweist:

   - eine Blutpumpe (1010), verbindbar mit einem extrakorporalen Blutkreislauf (3000) zum Fördern von Blut durch den extrakorporalen Blutkreislauf (3000);
   - eine Dialysierflüssigkeitszulaufleitung (1040);
   - eine Dialysierflüssigkeitspumpe (1210) zum Fördern von Dialysierflüssigkeit durch die Dialysierflüssigkeitszulaufleitung (1040) und/oder eine Ultrafiltrationspumpe und/oder eine Effluentpumpe;
   - eine Eingabevorrichtung (100) für den Anwender zum Eingeben von Flusswerten ($Q_B$, $Q_{dia}$, $Q_{UF}$), die während einer anstehenden Blutbehandlungssitzung mittels der Blutpumpe (1010) und/oder der Dialysierflüssigkeitspumpe (1210) und/oder der Ultrafiltrationspumpe und/oder der Effluentpumpe erzielt oder bewirkt werden sollen, und/oder zum Eingeben eines Behandlungsziels ($d_{targ}$), welches bei Abschluss der anstehenden Blutbehandlungssitzung erzielt sein soll;
   - eine Einlesevorrichtung (150) zum Einlesen und/oder Speichern der vom Anwender eingegebenen Flusswerte ($Q_B$, $Q_{dia}$, $Q_{UF}$) und/oder des vom Anwender eingegebenen Behandlungsziels ($d_{targ}$);
   - eine Steuer- oder Regelvorrichtung (1500), konfiguriert zum Steuern oder Regeln der Blutbehandlungsvorrichtung (1000) nach Beginn der anstehenden Blutbehandlungssitzung anhand von Flusswerten ($Q_B$, $Q_{dia}$, $Q_{UF}$) und/oder Behandlungsziele ($d_{targ}$) ;
   - eine Speichervorrichtung (160), in der ein Korrekturfaktor ($f_{korr}$) als Konstante hinterlegt ist;
   wobei die Steuer- oder Regelvorrichtung (1500) konfiguriert ist zum Ausführen eines Verfahrens, welches folgende Schritte umfasst:

a) Bereitstellen, Ermitteln oder Auslesen des Korrekturfaktors ($f_{korr}$) von oder aus der Speichervorrichtung (160);
b) Korrigieren der vom Anwender eingegebenen Flusswerte ($Q_B$, $Q_{dia}$, $Q_{UF}$) und/oder des vom Anwender eingegebenen
Behandlungsziels ($d_{targ}$) um den Korrekturfaktor ($f_{korr}$).

6. Blutbehandlungsvorrichtung (1000) nach Anspruch 5, wobei das Behandlungsziel ($d_{targ}$) ein Behandlungsergebnis bezogen auf die Behandlungsdauer ist.

7. Blutbehandlungsvorrichtung (1000) nach einem der Ansprüche 5 bis 6, wobei der Korrekturfaktor ($f_{korr}$) einen Wert zwischen 10 % und 30 %, insbesondere 20 % beträgt und/oder eine Korrektur der eingegebenen Flusswerte ($Q_B$, $Q_{dia}$, $Q_{UF}$) und/oder des vom Anwender eingegebenen Behandlungsziels ($d_{targ}$) zwischen 10 % und 30 %, insbesondere 20 % ergibt.

8. Blutbehandlungsvorrichtung (1000) nach einem der Ansprüche 5 bis 7, wobei der Korrekturfaktor ($f_{korr}$) mittels der Eingabevorrichtung (100) vom Anwender eingegeben wird oder wurde.

9. Blutbehandlungsvorrichtung (1000) nach einem der Ansprüche 5 bis 8, ausgestaltet als Hämodialysevorrichtung, Hämofiltrationsvorrichtung oder als Hämodiafiltrationsvorrichtung, insbesondere als eine Vorrichtung für die chronische Nierenersatztherapie oder für die kontinuierliche Nierenersatztherapie (CRRT = *continuous renal replacement therapy*).

10. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die maschinellen Schritte eines erfindungsgemäßen Verfahrens nach einem der Ansprüche 1 bis 4 veranlasst werden.

11. Computerprogramm-Produkt, als Signalwelle oder mit einem auf einem maschinenlesbaren Träger gespeicherten Programmcode, zur Veranlassung der maschinellen Schritte des erfindungsgemäßen Verfahrens gemäß einem der Ansprüche 1 bis 4, wenn das Computerprogramm-Produkt auf einem Rechner abläuft.

12. Computerprogramm mit einem Programmcode zur Veranlassung der maschinellen Schritte eines erfindungsgemäßen Verfahrens gemäß einem der Ansprüche 1 bis 4, wenn das Computerprogramm auf einem Computer abläuft.

**Claims**

1. A method of setting a control or regulating device (1500) of a blood treatment apparatus (1000) for an upcoming blood treatment session, the blood treatment apparatus comprising:

- a blood pump (1010), connectable to an extracorporeal blood circuit (3000) for conveying blood through the extracorporeal blood circuit (3000);
- a dialysis liquid inlet line (1040);
- a dialysis liquid pump (1210) for conveying dialysis liquid through the dialysis liquid inlet line (1040) and/or an ultrafiltration pump and/or an effluent pump;
- an input device (100) for the user for entering flow values ($Q_B$, $Q_{dia}$, $Q_{UF}$) to be achieved or effected during an upcoming blood treatment session, by means of the blood pump (1010) and/or the dialysis liquid pump (1210) and/or the ultrafiltration pump and/or the effluent pump, and/or for entering a treatment target ($d_{targ}$) to be achieved by completion of the upcoming blood treatment session;
- a reading device (150) for reading and/or storing the flow values ($Q_B$, $Q_{dia}$, $Q_{UF}$) entered by the user and/or the treatment target ($d_{targ}$) entered by the user;
- a control or regulating device (1500), configured for controlling or regulating the blood treatment apparatus (1000) after the start of the upcoming blood treatment session based on flow values ($Q_B$, $Q_{dia}$, $Q_{UF}$) and/or treatment targets ($d_{targ}$);
- a storage device (160), in which a correction factor ($f_{korr}$) is stored as a constant;
wherein the method encompasses the following steps:

a) providing, determining or reading out the correction factor ($f_{korr}$) from or out of the storage device (160);
b) correcting flow values ($Q_B$, $Q_{dia}$, $Q_{UF}$) entered by the user and/or the treatment target ($d_{targ}$) entered by the user by the correction factor ($f_{korr}$).

2. The method according to claim 1, wherein the treatment target ($d_{targ}$) is a treatment result relative to the duration of treatment.

3. The method according to anyone of the preceding claims, wherein the correction factor ($f_{korr}$) is a value between 10 % and 30 %, in particular 20 %, and/or results a correction of the entered flow values ($Q_B$, $Q_{dia}$, $Q_{UF}$) and/or of the treatment target ($d_{targ}$) entered by the user between 10 % and 30 %, in particular 20 %.

4. The method according to anyone of the preceding claims, wherein the correction factor ($f_{korr}$) is or has been entered by the user by means of the input device (100).

5. A blood treatment apparatus (1000), which comprises:

- a blood pump (1010), connectable to an extracorporeal blood circuit (3000) for conveying blood through the extracorporeal blood circuit (3000);
- a dialysis liquid inlet line (1040);
- a dialysis liquid pump (1210) for conveying dialysis liquid through the dialysis liquid inlet line (1040) and/or an ultrafiltration pump and/or an effluent pump;
- an input device (100) for the user for entering flow values ($Q_B$, $Q_{dia}$, $Q_{UF}$) to be achieved or effected during an upcoming blood treatment session, by means of the blood pump (1010) and/or the dialysis liquid pump (1210) and/or the ultrafiltration pump and/or the effluent pump, and/or for entering a treatment target ($d_{targ}$) to be achieved by completion of the upcoming blood treatment session;
- a reading device (150) for reading and/or storing the flow values ($Q_B$, $Q_{dia}$, $Q_{UF}$) entered by the user and/or the treatment target ($d_{targ}$) entered by the user;
- a control or regulating device (1500), configured for controlling or regulating the blood treatment apparatus (1000) after the start of the upcoming blood treatment session based on flow values ($Q_B$, $Q_{dia}$, $Q_{UF}$) and/or treatment targets ($d_{targ}$) ;
- a storage device (160), in which a correction factor ($f_{korr}$) is stored as a constant;
wherein the control or regulating device (1500) is configured for executing a method which encompasses the following steps:

a) providing, determining or reading out the correction factor ($f_{korr}$) from or out of the storage device (160);
b) correcting flow values ($Q_B$, $Q_{dia}$, $Q_{UF}$) entered by the user and/or the treatment target ($d_{targ}$) entered by the user by the correction factor ($f_{korr}$).

6. The blood treatment apparatus (1000) according to claim 5, wherein the treatment target ($d_{targ}$) is a treatment result relative to the duration of treatment.

7. The blood treatment apparatus (1000) according to anyone of claims 5 to 6, wherein the correction factor ($f_{korr}$) is a value between 10 % and 30 %, in particular 20 %, and/or results a correction of the entered flow values ($Q_B$, $Q_{dia}$, $Q_{UF}$) and/or of the treatment target ($d_{targ}$) entered by the user between 10 % and 30 %, in particular 20 %.

8. The blood treatment apparatus (1000) according to anyone of claims 5 to 7, wherein the correction factor ($f_{korr}$) is or has been entered by the user by means of the input device (100).

9. The blood treatment apparatus (1000) according to anyone of claims 5 to 8, designed as a hemodialysis apparatus, hemofiltration apparatus or hemodiafiltration apparatus, in particular as an apparatus for chronic renal replacement therapy or for continuous renal replacement therapy (CRRT = *continuous renal replacement therapy*).

10. A digital storage medium, in particular in form of a floppy disk, CD, or DVD or EPROM, with electronically readable control signals, configured to interact with a programmable computer system such that the automatic steps of a method based on the invention according to anyone of claims 1 to 4 are initiated.

11. A computer program product as a signal wave or with a programme code stored on a machine-readable carrier, for initiating the automatic steps of the method based on the invention according to anyone of claims 1 to 4, when the computer program product runs on a computer.

12. A computer program with a program code for initiating the automatic steps of the method based on the invention according to anyone of claims 1 to 4, when the computer program runs on a computer.

**Revendications**

1. Un procédé de réglage d'un dispositif de commande ou de régulation (1500) d'un appareil de traitement du sang (1000) pour une séance de traitement du sang à venir, l'appareil de traitement du sang comprenant:

- une pompe à sang (1010), pouvant être reliée à un circuit sanguin extracorporel (3000) pour acheminer le sang au travers du circuit sanguin extracorporel (3000);
- un conduit d'alimentation en liquide de dialyse (1040);
- une pompe à liquide de dialyse (1210) pour acheminer le liquide de dialyse au travers du conduit d'alimentation en liquide de dialyse (1040) et/ou une pompe d'ultrafiltration et/ou une pompe d'effluent;
- un dispositif de saisie (100) permettant à l'utilisateur d'entrer des valeurs de débit ($Q_B$, $Q_{dia}$, $Q_{UF}$) à atteindre ou à réaliser lors d'une séance de traitement du sang à venir, au moyen de la pompe à sang (1010) et/ou de la pompe à liquide de dialyse (1210) et/ou de la pompe d'ultrafiltration et/ou de la pompe d'effluent, et/ou d'entrer un objectif de traitement ($d_{targ}$) à atteindre à la fin de la séance de traitement du sang à venir;
- un dispositif de lecture (150) permettant de lire et/ou de sauvegarder des valeurs de débit ($Q_B$, $Q_{dia}$, $Q_{UF}$) entrées par l'utilisateur et/ou l'objectif de traitement ($d_{targ}$) entré par l'utilisateur;
- un dispositif de commande ou de régulation (1500), configuré pour commander ou réguler l'appareil de traitement du sang (1000) après le début de la séance de traitement du sang à venir sur la base de valeurs de débit ($Q_B$, $Q_{dia}$, $Q_{UF}$) et/ou d'objectifs de traitement ($d_{targ}$);
- un dispositif de stockage (160), dans lequel un facteur de correction ($f_{korr}$) est sauvegardé en tant que constante;
où le procédé comprend les étapes suivantes:

a) la fourniture, la détermination ou la lecture du facteur de correction ($f_{korr}$) depuis ou à partir du dispositif de stockage (160);
b) la correction des valeurs de débit ($Q_B$, $Q_{dia}$, $Q_{UF}$) entrées par l'utilisateur et/ou de l'objectif de traitement ($d_{targ}$) entré par l'utilisateur par le facteur de correction ($f_{korr}$).

2. Le procédé selon la première revendication, où l'objectif de traitement ($d_{targ}$) est un résultat de traitement basé sur la durée du traitement.

3. Le procédé selon l'une quelconque des revendications précédentes, où le facteur de correction ($f_{korr}$) est une valeur comprise entre 10 % et 30 %, notamment 20 %, et/ou entraîne une correction des valeurs de débit ($Q_B$, $Q_{dia}$, $Q_{UF}$) entrées et/ou de l'objectif de traitement ($d_{targ}$) entré par l'utilisateur entre 10 % et 30 %, notamment 20 %.

4. Le procédé selon l'une quelconque des revendications précédentes, où le facteur de correction ($f_{korr}$) est ou a été entré par l'utilisateur au moyen du dispositif de saisie (100).

5. Un appareil de traitement du sang (1000), qui comprend:

- une pompe à sang (1010), pouvant être reliée à un circuit sanguin extracorporel (3000) pour acheminer le sang au travers du circuit sanguin extracorporel (3000);
- un conduit d'alimentation en liquide de dialyse (1040);
- une pompe à liquide de dialyse (1210) pour acheminer le liquide de dialyse au travers du conduit d'alimentation en liquide de dialyse (1040) et/ou une pompe d'ultrafiltration et/ou une pompe d'effluent;
- un dispositif de saisie (100) permettant à l'utilisateur d'entrer des valeurs de débit ($Q_B$, $Q_{dia}$, $Q_{UF}$) à atteindre ou à réaliser lors d'une séance de traitement du sang à venir, au moyen de la pompe à sang (1010) et/ou de la pompe à liquide de dialyse (1210) et/ou de la pompe d'ultrafiltration et/ou de la pompe d'effluent, et/ou d'entrer un objectif de traitement ($d_{targ}$) à atteindre à la fin de la séance de traitement du sang à venir;
- un dispositif de lecture (150) permettant de lire et/ou de sauvegarder des valeurs de débit ($Q_B$, $Q_{dia}$, $Q_{UF}$) entrées par l'utilisateur et/ou l'objectif de traitement ($d_{targ}$) entré par l'utilisateur;
- un dispositif de commande ou de régulation (1500), configuré pour commander ou réguler l'appareil de traitement du sang (1000) après le début de la séance de traitement du sang à venir sur la base de valeurs de débit ($Q_B$, $Q_{dia}$, $Q_{UF}$) et/ou d'objectifs de traitement ($d_{targ}$);
- un dispositif de stockage (160), dans lequel un facteur de correction ($f_{korr}$) est sauvegardé en tant que constante;
où le dispositif de commande ou de régulation (1500) est configuré de manière à exécuter un procédé qui comprend les étapes suivantes:

a) la fourniture, la détermination ou la lecture du facteur de correction ($f_{korr}$) depuis ou à partir du dispositif de stockage (160);
b) la correction des valeurs de débit ($Q_B$, $Q_{dia}$, $Q_{UF}$) entrées par l'utilisateur et/ou de l'objectif de traitement ($d_{targ}$) entré par l'utilisateur par le facteur de correction ($f_{korr}$).

6. L'appareil de traitement du sang (1000) selon la revendication 5, où l'objectif du traitement ($d_{targ}$) est un résultat de traitement basé sur la durée du traitement.

7. L'appareil de traitement du sang (1000) selon l'une quelconque des revendications 5 à 6, où le facteur de correction ($f_{korr}$) est une valeur comprise entre 10 % et 30 %, notamment 20 %, et/ou entraîne une correction des valeurs de débit ($Q_B$, $Q_{dia}$, $Q_{UF}$) entrées et/ou de l'objectif de traitement ($d_{targ}$) entré par l'utilisateur entre 10 % et 30 %, notamment 20 %.

8. L'appareil de traitement du sang (1000) selon l'une quelconque des revendications 5 à 7, où le facteur de correction ($f_{korr}$) est ou a été entré par l'utilisateur au moyen du dispositif de saisie (100).

9. L'appareil de traitement du sang (1000) selon l'une quelconque des revendications 5 à 8, conçu comme appareil d'hémodialyse, appareil d'hémofiltration ou appareil d'hémodiafiltration, notamment comme appareil pour la thérapie de remplacement rénal chronique ou pour la thérapie de remplacement rénal continu (CRRT = *continuous renal replacement therapy*).

10. Un support de stockage numérique, notamment sous la forme d'une disquette, d'un CD, d'un DVD ou d'une EPROM, avec des signaux de commande lisibles électroniquement, configuré pour interagir avec un système informatique programmable de manière à déclencher les étapes automatiques du procédé fondé sur l'invention selon l'une quelconque des revendications 1 à 4.

11. Un produit de programme d'ordinateur sous la forme d'une onde de signal ou avec un code de programme sauvegardé sur un support lisible par machine, pour déclencher les étapes automatiques du procédé fondé sur l'invention selon l'une quelconque des revendications 1 à 4 lorsque le produit de programme informatique est exécuté sur un ordinateur.

12. Un programme d'ordinateur avec un code de programme pour déclencher les étapes automatiques du procédé fondé sur l'invention selon l'une quelconque des revendications 1 à 4 lorsque le programme informatique est exécuté sur un ordinateur.

Fig. 1

EP 3 765 116 B1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03028860 A1
- US 2016121037 A1
- WO 2013144793 A